# EUROPEAN PATENT SPECIFICATION

(11) **EP 0 238 304 B1**
(45) Date of publication and mention of the grant of the patent: **19.06.1996**
(21) Application number: 87302267.7
(22) Date of filing: 17.03.1987
(51) Int. Cl.: C12N 15/58, A61K 38/46

(54) **Modified human tissue-type plasminogen activator and its preparation**
Modifizierter, menschlicher Gewebeplasminogenaktivator und seine Herstellung
Activateur de plasminogène tissulaire humain modifié et sa préparation

(30) Priority: 18.03.1986 US 841075; 04.03.1987 US 21893
(43) Date of publication of application: 23.09.1987
(73) Proprietor: GENENTECH, INC., South San Francisco California 94080 (US)
(72) Inventor: Hotchkiss, Adair John, Half Moon Bay, CA 94019 (US); Spellman, Michael Walter, San Bruno, CA 94066 (US); O'Connor, John Vincent, San Carlos, CA 94070 (US)
(74) Representative: Armitage, Ian Michael

(56) References cited:
- EP-A- 0 178 105
- EP-A- 0 225 286
- EP-A- 0 227 462
- WO-A-84/01786
- WO-A-84/01960
- WO-A-87/04722
- DE-A- 3 537 176
- WO87/04722

## Description

### Field of the Invention

The present invention is directed to novel human tissue-type plasminogen activators characterized in that they are devoid of functional carbohydrate structure at amino acid residue 117. The novel human tissue-type plasminogen activators hereof are otherwise unmodified compared with native state, in functional carbohydrate structure at amino acid residues 184 and/or 448. Surprisingly the novel human tissue-type plasminogen activators hereof have retained substantially full biological activity, compared with otherwise native material, and have an unexpectedly increased in vivo half-life.

### Background of the Invention

Human tissue-type plasminogen activator converts plasminogen to plasmin. The plasmin, so produced, proteolytically cleaves fibrin matrices which comprise the backbone of blood clots. Human tissue-type plasminogen activator thereby mediates the dissolution of blood clots and is consequently useful in the treatment of various thrombolytic disorders.

The abbreviation t-PA for human tissue-type plasminogen activator was adopted after proposal at the XXVIII Meeting of the International Committee on Thrombosis and Hemostatis, Bergamo, Italy, 27 July 1982. As used herein, the terms "human tissue-type plasminogen activator", "t-PA" "human t-PA" or "tissue plasminogen activator" denote human extrinsic (tissue-type) plasminogen activator, produced, for example, from natural source extraction and purification [see Collen et al., European Patent Application No 41766 (published 16 December 1981 based upon a first filing of 11 June 1980) and Rijken et al., Journal of Biol. Chem. 256, 7035 (1981), incorporated herein by reference], and by recombinant cell culture systems as described together with its amino acid sequence and physical and biological characteristics, for example, in European Patent Application Publication No. 93619, (published 9 November 1983) based upon a first filing of 5 May 1982), incorporated herein by reference.

U.S. Patent No. 4326033 reports on extending the half-life of urokinase by chemically modifying its carbohydrate structure. Urokinase is immunologically distinct from human tissue-type plasminogen activator. There is no justification, either from U.S. 4326033 or otherwise, in considering that such carbohydrate modifications of urokinase would have applicability to other glycoproteins. Indeed, for example, removal of sialic acid from ceruloplasmin decreases its half-life dramatically; yet identical treatment of transferrin, another serum glycoprotein, has no significant effect on half-life. (Sharon, Complex Carbohydrates, Addison-Wesley Publ. Co., p. 194-196, (1975); see also Ashwell et al., Adv. Enzymologv 41, 99 (1974) and Alexander et al., Science 226, 1328 (1984).

In Patent Application International Publication No. W084/01786. published 10 May 1984 based upon a first filing of 28 October 1982, there is described an indiscriminate modification of tissue-type plasminogen activator resulting in a molecule with reduced biological activity and purported increased half-life, compared with the unmodified polypeptide. The single example involves treating a partially purified human tissue-type plasminogen activator with sodium periodate giving a product reported to have about 70 to 90 percent of the original (unmodified) activity. There is no indication in WO84/01786 of an appreciation of the nature and characterization of the carbohydrate structures present in native material, and more importantly, in the modified form they produce. In fact, periodate is known to modify or disrupt, by oxidation, all carbohydrate structures without their concomitant, substantial removal from amino acid linkage.

There is also no indication in WO84/01786 of how many such structures human tissue-type plasminogen activator has, or what the actual carbohydrate make-up is, either for unmodified or their modified version. Thus, their periodate treated molecule was most probably modified by oxidation of all carbohydrate structures indiscriminately, without focus on a particular site.

EP 227 462 discloses the production of t-PA mutants modified at glycosylation sites at both the 117-119 and 184-186 positions to change their specific activity.

EP 178 105 discloses the cloning of uterine t-PA, producing partially or non-glycosylated t-PA in eukaryotic cells.

Recently, it was reported that the in vivo clearance rates of glycosylated and deglycosylated human tissue-type plasminogen activator were not significantly different, forcing the conclusion that the clearance rate of human tissue-type plasminogen activator is not affected by the absence of carbohydrates (Larsen et al., Proteases in Biological Control and Biotechnology, UCLA Symposium Park City, Utah, February 9-14, 1986. See Little et al., Biochemistry 23, 6191 (1984).

### Summary of the Invention

Now it has been discovered that human tissue-type plasminogen activator has a specific carbohydrate structure at amino acid residue 117 that is considerably different from those carbohydrate structures at amino acid residues 184 and 448, and that when completely removed (or, apart from the N-acetylglucosamine moiety linked to the Asn amino acid) results in novel human tissue-type plasminogen activators having retained substantially full biological activity and an unexpectedly increased in vivo half-life.

The present invention is thus directed to a method of preparing human tissue-type plasminogen activators including treating t-PA with endoglycosidase to eliminate functional carbohydrate structure at amino acid residue 117, the tissue-type plasminogen activators otherwise having functionally unmodified carbohydrate structures (at amino acid residues 184 and/or 448), and having retained substantially full biological activity and increased in vivo half-life (compared with "native" human tissue-type plasminogen activator having an intact carbohydrate structure at amino acid residue 117).

The present invention is further directed to a method of preparing and the use of biologically active human tissue-type plasminogen activator equivalents differing in one or more amino acid(s) in the overall sequence but having the same carbohydrate pattern as the particular novel human tissue-type plasminogen activators hereof. The present invention is further directed to associated recombinant vectors, cultures, and methods useful for preparing the novel human tissue-type plasminogen activators hereof.

One such human tissue-type plasminogen activator equivalent included within the scope hereof is a so-called single-chain mutant wherein the cleavage site between amino acids 275 and 276 is destroyed by eliminating the amino acid sequence recognized by proteolytic enzymes. The elimination of the sequence is effected by changing specific amino acids, for example, by site-specific mutagenesis of the underlying DNA codons according to the method described infra.

### Figure Legends

Figure 1 represents a Comassie-stained SDS-PAGE of untreated (Lane 1) and Endo H treated (Lane 2) human tissue-type plasminogen activator. The high molecular weight band is 1-chain t-PA; the other major bands are type I Kringle (KI), type II Kringle (KII) and protease (P).

Figure 2 represents glycosidase digestions of reduced carboxymethylated t-PA. Lane 1: -N-glycanas; Lane 2: +N-glycanase; Lane 3: -Endo H; Lane 4: +Endo H. Band identifications as in Figure 1.

Figure 3 represents a restriction map of starting plasmid pUCPAΔHD.

Figure 4 represents a time course of the change of trichloroacetic acid (TCA) precipitable radioactivity for control human tissue-type plasminogen activator (■) and human tissue-type plasminogen activator that has been carried through the Endo H process without the enzyme (▲) and Endo H treated human tissue-type plasminogen activator (●). Data are mean +/- SD (n=5).

Figure 5 represents a time course of the change of trichloroacetic acid (TCA) precipitable radioactivity for control human t-PA (0) and glutamine ₁₁₇ t-PA (□).

Figure 6 represents a time course of the change of trichloroacetic acid (TCA) precipitable radioactivity for control human t-PA (□) and glutamine ₁₁₇ glutamic acid ₂₇₅ t-PA (0).

### Detailed Description

### A. General

It has been found that the carbohydrate structure at amino acid residue 117 of human tissue-type plasminogen activator has the following type of composition: 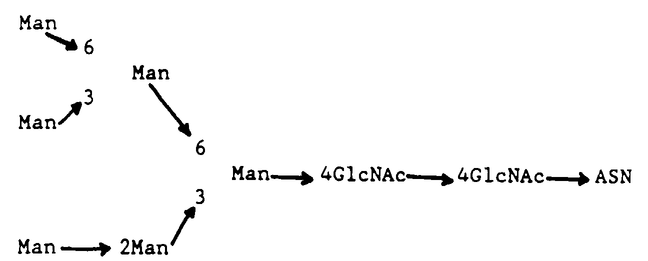 whilst the structures at amino acid residues 184 and 448 have the following types of compositions:

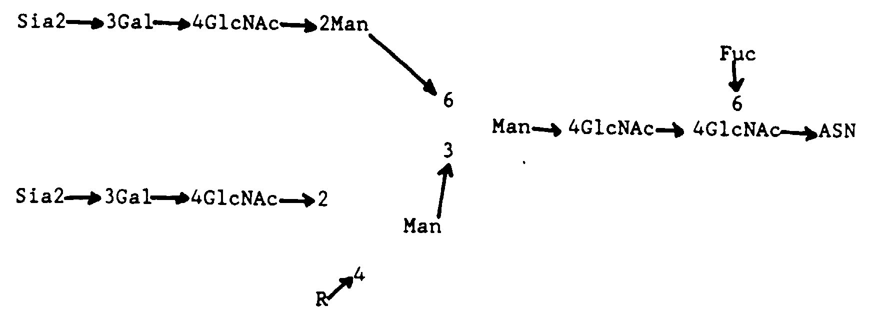

Abbreviations: Man- mannose; Gal- galactose; Fuc- fucose; GlcNAc- N. acetylglucosamine; Sia- sialic acid; R- H or Sia2→3Gal→4GlcNAc.

It should be emphasized that, while the above structures are representative of the types of N-linked oligosaccharides found on human tissue-type plasminogen activator, the present invention is not limited to the structures shown. Each glycosylation site probably contains several closely related, non-identical structures. This is known as microheterogeneity and is a common characteristic of glycoprotein glycans [See J. Biol. Chem. 260, 4046 (1985)]. For example, high-mannose oligosaccharides can vary in the number of mannose units present. In complex oligosaccharides, microheterogeneity can involve differences in the extent of branching as well as in the number of residues of sialic acid, fucose, galactose and N-acetylglucosamine. Such microheterogeneity is intended to be within the scope of the present invention.

The high-mannose containing structure at amino acid 117 proved unique, not only in structure from the more complex structures at amino acid residues 184 and 448, but also in that its complete functional removal without concomitant functional modification of the structures at 184 and 448 resulted in a fully biologically active human tissue-type plasminogen activator having increased in vivo half-life.

Removal of functional carbohydrate structure at amino acid residue 117 means complete removal, as where the glycosylation signal is destroyed by site-directed mutagenesis as described infra, or substantial removal, as by treatment with endoglycosidase which may leave an intact N-acetylglucosamine residue linked to Asn₁₁₇, for example. Functionally unmodified carbohydrate structure at amino acid residues 184 and/or 448 means either retention of the intact structure(s) or substantially all of such structure(s) such that they are functionally equivalent to native protein.

In the preferred embodiment, removal of functional carbohydrate structure at amino acid residue 117 is accomplished by site-specific mutagenesis of the underlying DNA of the glycosylation signal Asn-X-Ser/Thr, where X can be any amino acid. In the case of tissue-type plasminogen activator, the sequence representing this signal is Asn₁₁₇ (Ser₁₁₈) Ser₁₁₉. Removal of functional carbohydrate structure at amino acid residue 117 thus results, for example, by mutagenizing the codons corresponding to these amino acid residues, destroying the signal functionality. In particular, mutagenesis can be performed on representative codons of the signal such that there is produced, for example, a human tissue-type plasminogen activator having an amino acid residue other than asparagine (Asn) at position 117 and/or other than serine (Ser) or threonine (Thr) at position 119 or a proline (Pro) at position 118. In the most preferred embodiments, asparagine ₁₁₇ is replaced with glutamine, in view of their close structural similarity, or serine₁₁₉ is replaced with methionine, in view of an analogous sequence in the second kringle region.

The mutagenesis is accomplished via techniques known per se, for example, according to the methods reviewed by Zoller et al., Methods in Enzymology 100, 468 (1983). For example, by changing the asparagine encoding codon AAC at position 117 with CAA or CAG, requiring two nucleotide changes, the expression product will contain glutamine at position 117. Other mutations contemplated herein follow from analysis of the genetic code.

An alternative method of functional carbohydrate removal at amino acid residue 117 involves the use of an endoglycosidase such as Endoglycosidase H which is capable of (substantially) removing the high mannose carbohydrate structure at amino acid residue 117 (Asn) without functionally affecting the complex structures at amino acid residues 184 and 448. Again, this treatment is accomplished via techniques known per se, for example, according to the method of Tarentino et al., J. Biol. Chem. 249, 811 (1974) and Trimble et al., Anal. Biochem. 141. 515 (1984).

### B. Examples

### Example 1

Site specific mutagenesis was used to construct an expression vector operably harboring DNA encoding tissue-type plasminogen activator having amino acid residue glutamine at position 117, as follows:

### A. Oligonucleotide Design

A 24-mer oligonucleotide having the sequence 5'-TCC-ACC-AAC-TGG-C*A*A*-AGC-AGC-GCG-3ʹ (24-mer Q117) ws synthesized by the phosphotriester method of Crea et al., Nucleic Acids Research 8, 2331 (1980). Asterisks indicate the mutant (asn to gln) codon.

### B. Construction of Recombinant M13 Template

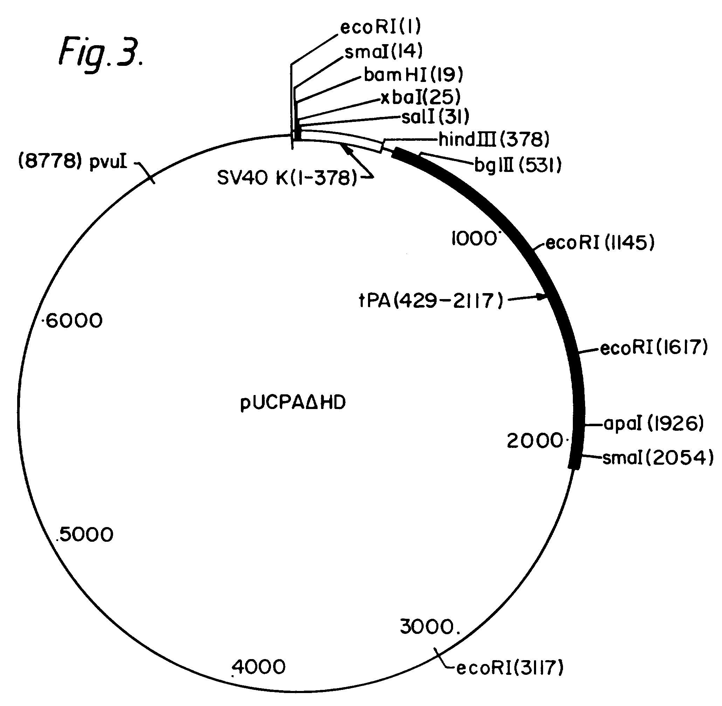

Plasmid pUCPAΔHD (Figure 3) is a derivative of plasmid designated pETPFR (otherwise designated pPADHFR-6 disclosed in EPO 93619, supra.), with the following modifications: 1) 166 b. p. of 5ʹ untranslated DNA has been trimmed from the 5ʹ end of the t-PA gene, using exonuclease Bal 31; 2) a Hind III site has been added to the new 5ʹ end of the t-PA gene; 3) a polylinker, containing recognition sites for EcoRl, Sac I, Sma I, Bam HI, Xba I, Sal I, and Pvu II, has been added to the 5ʹ end of the SV40 early promoter that drives t-PA expression; 4) the Hind III site at position 3539 of pETPFR has been destroyed by a Klenow fill-in reaction.

Plasmid pUCPAΔHD (Figure 3) was digested with SmaI, and the ca. 2.0 kb fragment containing the t-PA gene through codon No. 507 was isolated by PAGE and electroelution of the fragment from the gel. M13mplO (Messing, Methods in Enzymology 101, 20 (1983) vector was also digested with SmaI, extracted once with phenol, chloroform, ethanol precipitated, and resuspended in 50 mMtris pH8.0, lmMEDIA (TE). The ca. 2.0 kb fragment from pUCPAΔHD was ligated into the SmaI cut M13mplO using T4 DNA ligase and the resulting DNA was used to transform E. coli JMlOl. The resulting phage was isolated and the presence of the insert was verified and its orientation determined by restriction analysis of phage mini-preps. One recombinant phage, M13/t-PA-SMA, was chosen as template for subsequent mutagenesis.

### C. Mutagenesis Reaction

The mutagenesis primer (24-mer Q117) was annealed to single-stranded M13/t-PA-SMA DNA, and treated with E. coli DNA polymerase Klenow fragment in the presence of dNTPs and T4 DNA ligase to create in vitro heterodoplex RF molecules, as described by Adelman et al., DNA 2, 183 (1983). These molecules were used to transform E. coli strain JMlOl (ATCC No. 33876) and phage incorporating the desired mutation were detected by plague hybridation using the mutagenesis primer as a probe. (Adelman et al., DNA 2, 183 (1983). One mutant phage was isolated and designated M13/t-PA-0SMA-GLN117.

### D. Subcloning the GLN117 t-PA Mutant into Expression Plasmid pUCPAΔHD

Double stranded DNA of Phage M13/t-PA-SMA-GLN117 was digested with SmaI, Bg111 and ApaI and the ca 1.4 kb fragment purified by PAGE. This fragment was then used to replace the corresponding fragment in pUCPAΔHD.

Recombinant plasmids containing the t-PA gene fragment were identified. Plasmids M119 snd Q117 are introduced into and amplified in DHFR deficient CHO cells (Urlab et al., Proc. Natl. Acad. Sci. 77, 4216 (1980) as follows: 1) plasmid DNA is introduced into the cells by the calcium phosphate precipitation method of Graham et al., J. Virol. 52, 455 (1973); 2) colonies arising in selective medium [medium lacking hopoxanthine, glycine, and thymidine (-HGT) are assayed for t-PA expression indirectly by detecting plasmin formation as assessed by the digestion of fibrin in an agar plate containing fibrin and plasminogen, described by Granellia et al., J. Exp. Med. 148, 223 (1978); 3) five of the most strongly positive clones are assayed quantitatively for the amount of t-PA secreted per cell using an ELISA assay; 4) the clone secreting the highest level of t-PA is plated into methotrexate (MTX) as follows: 2 × 10⁵ cells are plated into 100 mm plates containing 50, 100, or 250 nM MTX; 5) five clones arising in MTX are extracted and assayed quantitatively (by ELISA) as in Step 3) above; 6) the clone secreting the highest level of t-PA is plated into higher concentrations of MTX as in Step 4) above, followed by quantitative assay of five clones that arise, and selection of the highest t-PA producer.

The above amplification and screening procedure is repeated until no increases in t-PA production are obtained from the resulting cell line and the corresponding mutant t-PA is separated for use.

### Example 2

A similar procedure as that described in Example 1 was used to make the corresponding Met₁₁₉ mutant using a 24-mer with the sequence 5ʹ-CC-AAC-TGG-AAC-AGC-A*T*G*-GCG-TTG-G-3ʹ (24-mer M119) to give pUCPAΔHD M119. Production of the corresponding M119 mutant by expression is as described above in Example 1.

### Example 3

A glutamine ₁₁₇ glutamic acid ₂₇₅ t-PA mutant was prepared as follows:

Plasmid pUCPAΔHD, prepared as described above, was digested with Bgl II and Sca I and the about 763 bp fragment, corresponding to codons 1 to 254 of the tissue-type plasminogen activator DNA sequence, was purified on SDS-PAGE in a manner known per se.

Human t-PA DNA was obtained from plasmids pPADHFR-6 (also designated pETPFR) and pA25E10. The preparation of these two t-PA plasmids is described in European Patent Application Publication No. 093619, referred to above and incorporated herein by reference.

Plasmid pA25E10 contains sequences coding for the last 508 amino acids of the t-PA gene and 772 base pairs of the 3ʹ untranslated region. This plasmid was digested with SacI and BglII to produce a 744 base pair fragment which was isolated by standard methods as previously described. This fragment contains the codons for t-PA amino acids 411 through 527 and includes part of the 3ʹ untranslated region.

Plasmid pPADHFR-6 contains the entire structural gene for t-PA and part of the 3ʹ untranslated region. This plasmid was digested with SacI and BglII to produce a 1,230 base pair fragment which was isolated. This fragment contains codons for the first 410 amino acids of the mature form of t-PA.

These fragments were ligated together using standard methods and digested with BglII. A 1,974 base pair fragment containing codons for the entire mature t-PA sequence plus part of the 3ʹ untranslated region was isolated. Double stranded MI3mp8, [Messing et al., Third Cleveland Symposium on Macromolecules Recombinant DNA, Editor A. Walter, Elsevier, Amsterdam (1981), p. 143] was digested with BamHI and annealed to the BglII digested t-PA to form M13mp8PABglII. E. coli JM 101 cells (ATCC No. 33876) were transformed with the double stranded replicative form of M13mp8PABglII. The single stranded and double stranded (RF) forms of M13mp8PABglII may be isolated from E. coli JM 101 cells infected with this phage. The single stranded form was used for the site specific mutagenesis of t-PA.

The human t-PA structural gene was modified by site specific mutagensis to express t-PA with amino acid substitutions at various positions. A synthetic oligonucleotide was prepared such as by the solid phase phosphotriester method of Crea et al., Proc Nati. Acad. Sci. (USA) 75, 5765 (1978) and used for such site specific mutagenesis:

Primer 2C9
                                        Glu
DNA Sequence    G  CCT  CAG  TTT  GAA  ATC  AAA  GGA  G

The general method of Adelman et al., DNA 2, 183 (1983). incorporated herein by reference, was used to generate a t-PA clone containing the mutated sequence of the synthetic primer. Mutant t-PA clone M13RF2C9 was generated by the use of the primer containing the mutation for the single amino acid shown above.

In the plasmid pPADHFR-6 (also designated pETPFR - see European Patent Application Publication No. 93619 supra) the expression of the native t-PA structural gene is under the control of the early promoter for SV40 T-antigen. This promoter also controls the expression of the DHFR gene. A vector Fragment 1 was obtained by isolating the large fragment generated by digestion of pPADHFR-6 with BglII and BstEII. Another Fragment 2 was obtained by isolating the 400 base pair t-PA fragment obtained from the digestion of pPADHFR-6 with BglII and BstXI. A 1,141 base pair t-PA Fragment 3 containing the desired mutation was obtained by digesting RF DNA from mutant t-PA clone M13RF2C9 with BstXI and BstEII. Fragments 1 and 2 were ligated with Fragment 3. The DNA mixture was used to transform E. coli to give eukaryotic expression vector pPADHFR-6 2C9.

Plasmid pPADHFR-6 2C9, prepared as described above and in European Patent Application Publication No. 199574, published 29 October 1986, contains a DNA sequence encoding glutamic acid ₂₇₅ tissue-type plasminogen activator mutant. It was digested with Sca I and Apa I and the about 630 bp fragment, corresponding to codons 254 to 466 of the tissue-type plasminogen activator DNA sequence, was purified on SDS-PAGE in a manner known per se.

The two BglII-ScaI (pUCPAΔHD) and ScaI-ApaI (pPADHFR-6 2C9) fragments were ligated into the large Bgl II (bp 531) Apa I (1926 bp) fragment from digested pUCPAΔHD and the resultant plasmid harboring glutamine ₁₁₇ glutamic acid ₂₇₅ t-PA mutant DNA was miniscreened in the usual manner. The resultant plasmid was introduced into and amplified in DHFR deficient CHO cells as described above and the corresponding mutant t-PA was separated for use.

### Example 4

The amino acid sequence of t-PA includes 4 potential N-linked glycosylation sites [Asn-X-Ser/Thr; Ann. Rev. Biochem. 41, 673 (1972)]. These are asparagine residues 117, 184. 218 and 448 [Nature 301, 214 (1983)]. Position 218, however, has been found not to be glycosylated in t-PA. Position 184 is glycosylated in type I t-PA but not in type II t-PA [Biochemistry 23, 3701 (1984)].

Gel-filtration chromatography of Pronase-digested rt-PA resolved two classes of N-linked oligosaccharides (Table 1). The composition of the higher molecular weight material was consistent with fucosylated complex-type oligosaccharides. The lower molecular weight material had the expected composition for a small high-mannose oligosaccharide (probably Man₆GlcNAc₂).

The attachment position of the high-mannose oligosaccharide was determined by utilizing glycosidic enzymes of different specificities. The enzymes used were endo-β-N-acetylglucosaminidase H (Endo H; Genzyme, Inc.), that removes high-mannose oligosaccharides but has no effect on complex-type oligosaccharides, and peptide-N-glycosidase F (N-glycanase; Genzyme, Inc.), that removes both high-mannose and complex-type oligosaccharides. The t-PA used for these experiments had been converted to the two-chain form with plasmin and then reduced and carboxymethylated.

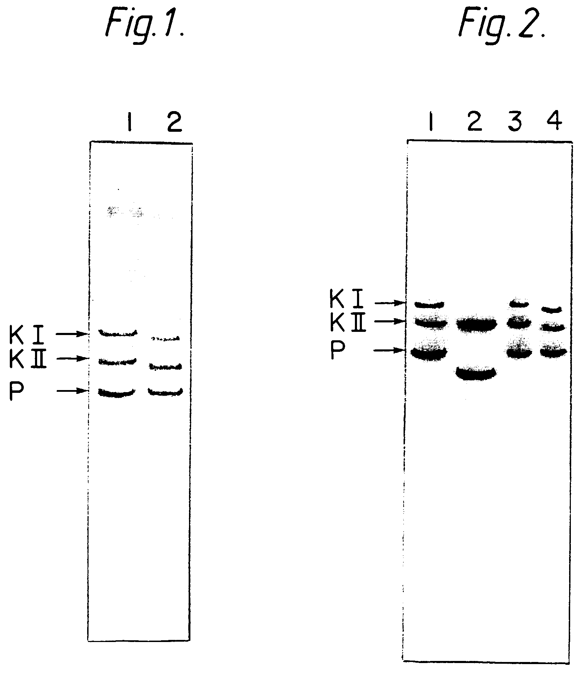

SDS-PAGE resolves reduced carboxymethylated two-chain rt-PA into type I kringle (glycosylation at 117 and 184), type II kringle (glycosylation at 117) and protease (glycosylation at 448). N-glycanase digestion of t-PA causes the kringle bands to coalesce at a position of slightly greater mobility than type II kringle and also causes increased mobility of the protease (lane 2, Fig. 2). Endo H digestion of t-PA increases the electrophoretic mobility of each kringle band, but does not affect the mobility of the protease band (lane 4, Fig. 2). The endo H result indicates that type I and type II kringle each contain a high-mannose oligosaccharide; this must be located at residue 117, which is the only position glycosylated in both type I and type II kringles. Endo H treatment does not convert type I to type II kringle; therefore, residue 184, which is glycosylated in type 1 but not in type 11 kringle, contains a complex oligosaccharide. Position 448 must also contain a complex structure, because N-glycanase treatment increases the mobility of the protease portion of rt-PA, while endo H has no effect.

**Table I**

| Carbohydrate Composition of Oligosaccharide Fractions From Pronase-Digested t-PA^{a} | | | | | |
|---|---|---|---|---|---|
| Sample | Fuc | Man | Gal | GlcNAc | Sia^{b} |
| Complex Type^{c} | 1.0 | 3.0 | 2.8 | 4.2 | 2.4 |
| High-mannose Type^{d} | 0.6 | 6.2 | trace | 2.0 | 0 |

| | | | | | |
|---|---|---|---|---|---|
| ^{a}Abbreviations: Fuc = Fucose, Man = Mannose, Gal = Galactose GlcNAc = N-acetylglucosamine, Sia = sialic acid | | | | | |
| ^{b}Thiobarbituric acid assay | | | | | |
| ^{c}Normalized to 3 Mannose | | | | | |
| ^{d}Normalized to 2 GlcNAc | | | | | |

### Example 5

Endo-β-N-acetylglucosaminidase H (Endo H) was purchased from Genzyme. Incorporated. SDS-PAGE was performed as described by Laemmli, Nature 227, 680 (1970). 0.8 mg of human tissue-type plasminogen activator (prepared as described in EPA 93619, supra.) (in 0.2 ml formulation buffer consisting of 0.2 M arginine phosphate, pH 6, containing 0.01% Tween 80) was mixed with Endo H (0.1 unit in 0.05 ml of 25 mM sodium phosphate, pH 6) and sodium azide (0.02 percent). The sample was incubated at 37 degrees for 20 hours. A control human tissue-type plasminogen activator sample was prepared and incubated in the same manner except that sodium phosphate buffer (0.05ml of 25 mm) was substituted for the Endo H solution. After incubation, the samples were diluted to a total volume of 0.75 ml with formulation buffer and extensively dialyzed into the same formulation buffer. The samples were filtered (0.4 micron HV filters, Amicon) and stored at 4 degrees.

Deglycosylation was monitored by SDS-PAGE after reduction and carboxymethylation. Aliquots of the thus prepared human tissue-type plasminogen activator (0.05 mg in 0.01 ml formulation buffer) were mixed with 25 mM sodium phosphate pH 6 (0.015 ml) and 2x Laemmli sample buffer containing 20 mM dithiothreitol (0.025 ml). The samples were heated for 5 minutes at 95 degrees and allowed to cool. Iodoacetic acid (0.015 ml of a 0.67 M solution in 1N NH₄OH) was added and the samples incubated in the dark for 3 hours at room temperature. The reduced carboxymethylated samples were analyzed by SDS-PAGE.

In this analysis, untreated control 2-chain human tissue-type Plasminogen activator is resolved into three major bands, corresponding to type I kringle (glycosylation at positions 117 and 184), type II kringle (glycosylation at position 117), and protease (lane 1, Fig. 1). Endo H digestion of human tissue-type plasminogen activator increases the electrophoretic mobility of each kringle band, but does not affect the mobility of the protease band (lane 2, Fig. 1).

The fibrinolytic activity of Endo H-treated human tissue-type plasminogen activator was assayed by the in vitro clot lysis assay of Collen et al., J. Clin. Path. 21, 705 (1968). The activity of Endo H-treated human tissue-type plasminogen activator was indistinguishable from that of the untreated control in this assay.

The human tissue plasminogen samples were iodinated by the Iodobead procedure [Markwell, Anal. Biochem. 125, 427 (1982)] to a specific activity of approximately 2 µCi/µg. Arginine, 0.2 M, and citrate, 0.1 M, (pH 6.0) and Tween 80, 0,01 percent, was the buffer used at all times. All samples were dialyzed into this buffer prior to iodination. pH was adjusted to 8.2 with Tris base prior to iodination. The iodination mixture was passed across a PD.1O column (Pharmacia) equilibrated with pH 6.0 buffer, the radioactive fractions from the void volume were pooled, SDS-PAGE was run and the dried gel was autoradiographed. Autoradiography of the labeled human tissue-type plasminogen activators showed that more than 95 percent of the radio-activity was incorporated into human tissue-type plasminogen activator.

Each labeled human tissue-type plasminogen activator was mixed with unlabeled material in a ratio of 1:200 (labeled:unlabeled, w/w) and injected i.v. as a bolus into rabbits that had an arterial catheter in the ear. Each rabbit received 1 mg/kg of unlabeled and 10 µCi/kg of labeled human tissue-type plasminogen activator. The unlabeled human tissue-type plasminogen activator was used as a carrier for the trace amount of labeled human tissue-type plasminogen activator in order to achieve therapeutic levels and to avoid alterations in the pharmacokinetics that could arise from concentration dependence in the clearance pathways. Serial arterial blood samples were collected over a 26 minute period and placed immediately into tubes containing a lyophilized mixture of D-phe-pro-arg-chloromethylketene (PPACK) and EDTA at final concentrations of 1µM and 4.8 mM, respectively. The tubes were placed on ice and plasma was separated. Trichloroacetic acid (TCA) precipitable (intact human tissue-type plasminogen activator) and total radioactivity were measured in each plasma sample. The immunoreactive human tissue-type plasminogen activator was also measured by a sandwich ELISA procedure which utilized polyclonal antibodies and had an effective sensitivity of at least 30 ng/ml.

Two types of data were generated from in vivo clearance studies in rabbits. One is from the immunoreactive human tissue-type plasminogen activator which should be a measure of the clearance of the unlabeled material. The second type of data is the TCA precipitable radioactivity which represents more than 95 percent intact human tissue-type plasminogen activator. The curves of plasma concentration versus time from the immunoreactivity and the TCA precipitable counts were fit to the appropriate multiexponential models and the derived pharmacokinetic parameters were compared.

### Example 6

### Pharmacokinetics of Endo H-treated Human Tissue-type Plasminogen Activator

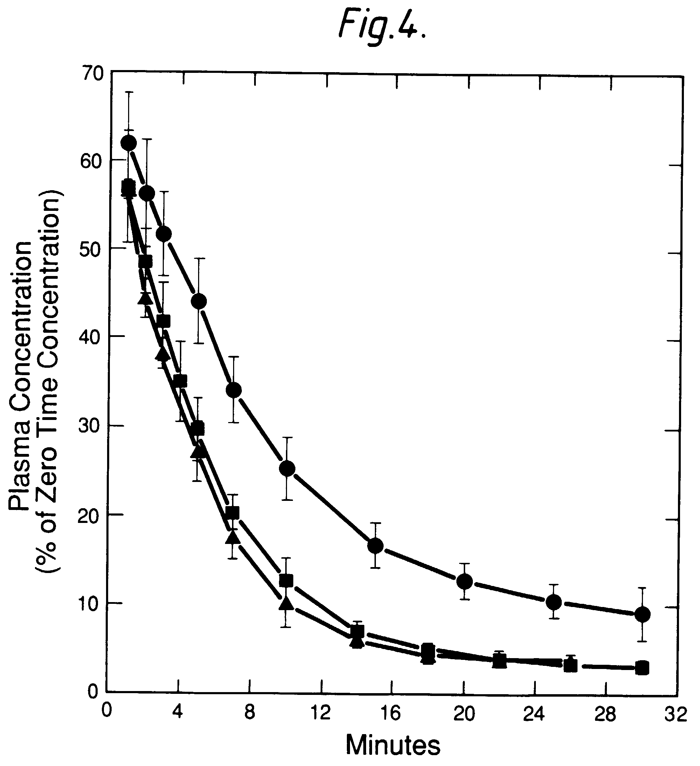

The pharmacokinetic profiles of the Endo H-treated human tissue-type plasminogen activator and control human tissue-type plasminogen activator and a second control of human tissue-type plasminogen activator which had been carried through the same reaction conditions as the Endo H-treated human tissue-type plasminogen activator in the absence of the enzyme are shown in Figure 4. The substantially identical profiles of the two controls show that the manipulations that were necessary to remove the simple sugar at 117 were not contributing to the change in clearance of human tissue-type plasminogen activator. The Endo H-treated human tissue-type plasminogen activator.is cleared much more slowly. Analysis of the data indicates that there is an increase in the alpha phase clearance rate in addition to an increase in the zero time extrapolated concentration for the beta phase. The Endo H-treated human tissue-type plasminogen activator has increased bioavailability as measured by the integral of the product of time and concentration; this is a relatively assumption free measure of bioavailability which is called the area under the curve, increased by a factor of about 2 by Endo H treatment.

Each test group received a carrier dose of unlabeled human tissue-type plasminogen activator. In all cases the pharmacokinetic profiles of the unlabeled human tissue-type plasminogen activator were not different for any group tested. This acts as an internal control to verify that the rabbits which were chosen for any given group did not by chance have unusual clearance characteristics.

### Pharmacokinetics of Gln₁₁₇ t-PA and Gln₁₁₇ Glu₂₇₅ t-PA Mutants

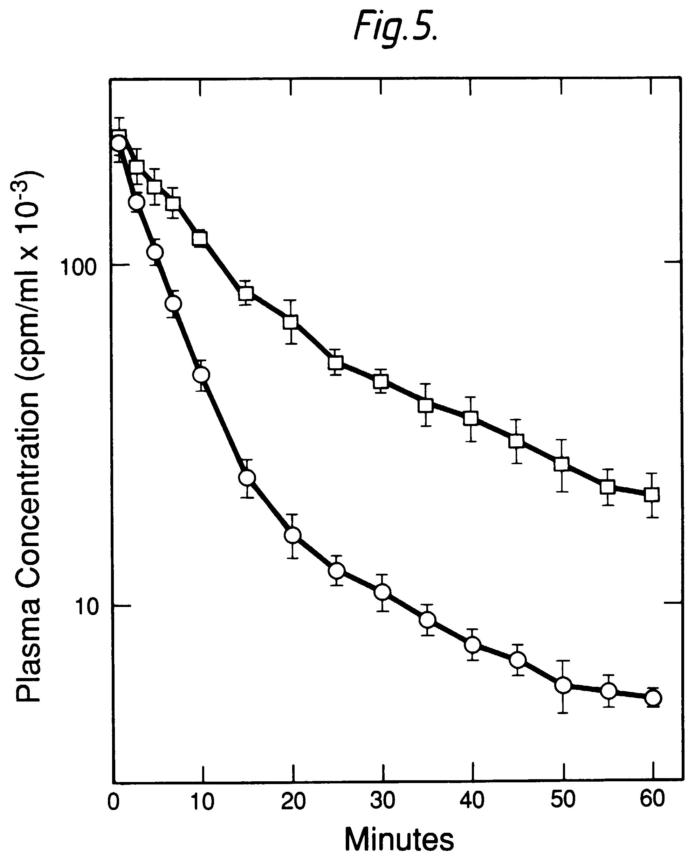

The pharmacokinetics of glutamine₁₁₇ t-PA and control human t-PA are shown in Figure 5. The glutamine₁₁₇ t-PA is cleared much more slowly than the control.

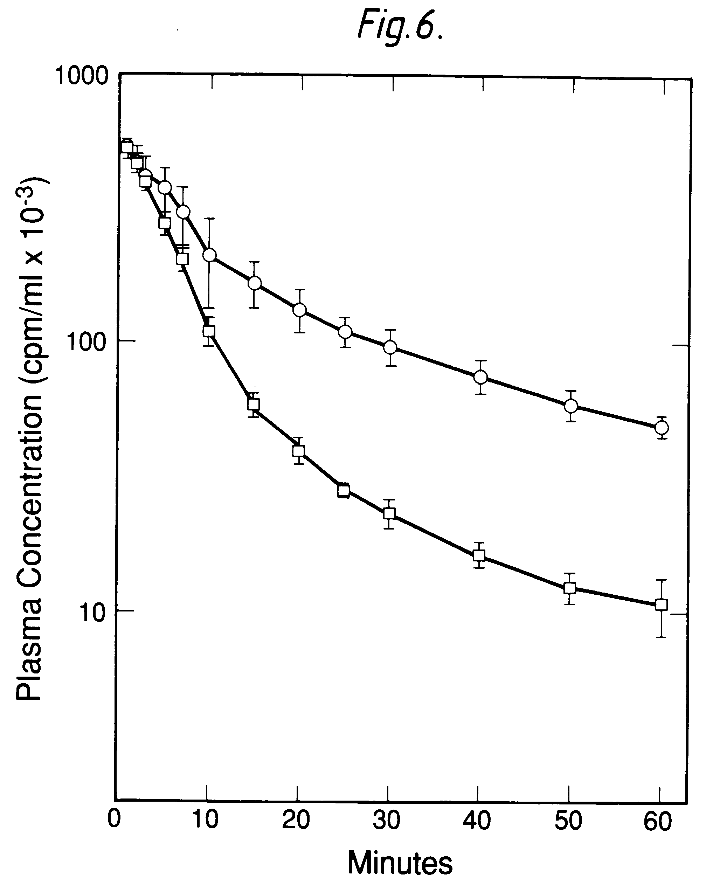

A similar profile is demonstrated by the glutamine₁₁₇ glutamic acid₂₇₅ t-PA, prepared as described in Example 3, as shown in Figure 6.

### Fibrin Binding Characteristics

Fibrin binding is an extremely important factor which is presumably directly related to the fibrin specificity which human tissue-type plasminogen activator possesses in vivo. Fibrin binding of the Endo H human tissue-type plasminogen activator was evaluated by two procedures. The first procedure utilized the capture of human tissue-type plasminogen activator by fibrin coated on a well in a standard microtiter dish; each well is then washed and a solution of plasminogen and a chromogenic substrate for plasmin (S-2251, Kabi) is added. The color which is generated is proportional to the amount of human tissue-type plasminogen activator which is captured in the initial step (Angles-Cano, Thrombosis and Haemostasis 54, 171 (1985). The second type of assay for fibrin binding measures the quantity of human tissue-type plasminogen activator which is left in solution (by ELISA) when thrombin is added to a solution of plasminogen-free fibrinogen and human tissue-type plasminogen activator (Rijken et al, J. Biol. Chem. 275, 2920 (1982). It is presently unclear which assay adequately predicts the in vivo consequences of altered human tissue-type plasminogen activator fibrin binding. Based upon the data from each assay, we can conclude that Endo H-treated human tissue-type plasminogen activator has at least unchanged if not improved fibrin specificity.

Similarly, fibrin binding test data of glutamine₁₁₇ glutamic acid₂₇₅ t-PA, of Example 3, demonstrated that the glutamine₁₁₇ glutamic acid₂₇₅ t-PA is similar to glutamic acid₂₇₅ t-PA and is superior to t-PA control in fibrin stimulation and specific activity.

### Example 7

The gln₁₁₇ and met₁₁₉ and mutants prepared as described in Examples 1 and 2 are each tested for fibrinolytic activity with similar results as with Endo H-treated material, as described above. The pharmacokinetics of each also are similar to those of Endo H-treated material, as compared with control human tissue-type plasminogen activators, as described above.

### Example 8

### Pharmaceutical Compositions

The compounds of the present invention can be formulated according to known methods to prepare pharmaceutically useful compositions, whereby the human tissue-type plasminogen activator product hereof is combined in admixture with a pharmaceutically acceptable carrier vehicle. Suitable vehicles and their formulation, inclusive of other human proteins, e.g., human serum albumin, are described for example in Remington's Pharmaceutical Sciences by E.W. Martin, which is hereby incorporated by reference. Such compositions will contain an effective amount of the protein hereof together with suitable amount of vehicle in order to prepare pharmaceutically acceptable compositions suitable for effective administration to the host.

For example, the human tissue-type plasminogen activator hereof may be parenterally administered to subjects suffering from cardiovascular diseases or conditions. Dosage and dose rate may parallel that currently in use in clinical investigations of other cardiovascular, thrombolytic agents, e.g. about 1.2 mg/kg body weight as an intravenous or intra-arterial dose over 1-5-12 hours in patients suffering from myocardial infarction, pulmonary embolism, etc.

As one example of an appropriate dosage form, a vial containing 50 mg human tissue-type plasminogen activator, arginine, phosphoric acid and polysorbate 80 may be reconstituted with 50 ml sterile water for injection and mixed with a suitable volume of 0.9 percent Sodium Chloride Injection.

The extended half-life of human tissue-type plasminogen activator may be suitable for rapid i.v. injection. This would eliminate the need for complex administration procedures and may increase the opportunity for the use of t-PA in settings with limited medical equipment such as in emergency vehicles staffed with paramedic personnel. An extended half-life of human tissue-type plasminogen activator may also allow lower, safer initial doses and could maintain thrombolytically effective plasmin levels for up to 45 minutes or longer. 'A longer half-life of human tissue-type plasminogen activator may also be useful for low dose extended therapy which may be necessary to avoid reocclusion following successful acute thrombolysis or for extended thrombolysis which may be necessary in cases of peripheral vascular occlusion.

## Claims (Claims for the following Contracting State(s): BE, CH, DE, FR, GB, IT, LI, LU, NL, SE)

1. A method of preparing human tissue-type plasminogen activator (t-PA) having retained substantially full biological activity and increased in vivo half-life compared to native t-PA, which includes treating t-PA with endoglycosidase which eliminates the functional carbohydrate structure which occurs at amino acid residue 117 in native t-PA, but which retains functionally unmodified carbohydrate structures at amino acid residues 184 and 448.

2. A method according to claim 1 wherein the t-PA is in single-chain form.

3. A method according to claim 2 wherein the amino acid sequence is mutated to destroy the cleavage site between amino acid residues 275 and 276.

4. A method according to claim 3 wherein the t-PA has a glutamic acid residue at position 275.

5. The use in the preparation of a pharmaceutical composition of human t-PA (a) having a mutation which destroys the glycosylation site only at amino acid residue 117-119, and (b) optionally having a mutation to eliminate the cleavage site at amino acid residues 275-276, wherein the composition retains substantially full biological activity and has increased in vivo half-life compared to a composition of native t-PA.

6. The use in the preparation of a pharmaceutical composition of human t-PA (a) devoid of functional carbohydrate structure at amino acid residue 117, (b) otherwise having functionally unmodified carbohydrate structures at amino acid residues 184 and 448, and (c) optionally having a mutation to eliminate the cleavage site at amino acid residues 275-276, wherein the composition retains substantially full biological activity and has increased in vivo half-life compared to a composition of native t-PA.

7. The use according to claim 5 or claim 6 wherein the t-PA contains an amino acid other than asparagine at position 117.

8. The use according to claim 5 or claim 6 wherein the t-PA contains an amino acid other than serine or threonine at position 119.

9. The use according to claim 5 or claim 6 wherein the t-PA contains glutamine at position 117.

10. The use according to claim 5 or claim 6 wherein the t-PA contains methionine at position 119.

11. The use according to claim 5 or claim 6 wherein the t-PA contains proline at position 118.

12. The use according to any one of claims 5 to 11 wherein the t-PA is in single chain form.

13. The use according to claim 12 wherein the t-PA has glutamic acid at position 275.

14. The use according to claim 12 wherein the t-PA contains glutamine at position 117 and glutamic acid at position 275.

15. The use according to any one of claims 5 to 14 wherein the t-PA is produced via recombinant DNA expression of recombinant DNA encoding said human t-PA having a codon at position 117 other than one encoding the amino acid asparagine or a codon at position 119 other than one encoding the amino acid serine or threonine.

16. A t-PA pharmaceutical composition having retained substantially full biological activity and increased in vivo half-life, comprising human t-PA as defined in any one of claims 1 to 15 in admixture with a pharmaceutically acceptable carrier.

## Claims (Claims for the following Contracting State(s): AT, ES, GR)

1. A method of preparing human tissue-type plasminogen activator (t-PA) having retained substantially full biological activity and increased in vivo half-life compared to native t-PA, which includes treating t-PA with endoglycosidase which eliminates the functional carbohydrate structure which occurs at amino acid residue 117 in native t-PA, but which retains functionally unmodified carbohydrate structures at amino acid residues 184 and 448.

2. A method according to claim 1 wherein the t-PA is in single-chain form.

3. A method according to claim 2 wherein the amino acid sequence is mutated to destroy the cleavage site between amino acid residues 275 and 276.

4. A method according to claim 3 wherein the t-PA has a glutamic acid residue at position 275.

5. The use in the preparation of a pharmaceutical composition of human t-PA (a) having a mutation which destroys the glycosylation site only at amino acid residue 117-119, and (b) optionally having a mutation to eliminate the cleavage site at amino acid residues 275-276, wherein the composition retains substantially full biological activity and has increased in vivo half-life compared to a composition of native t-PA.

6. The use in the preparation of a pharmaceutical composition of human t-PA (a) devoid of functional carbohydrate structure at amino acid residue 117, (b) otherwise having functionally unmodified carbohydrate structures at amino acid residues 184 and 448, and (c) optionally having a mutation to eliminate the cleavage site at amino acid residues 275-276, wherein the composition retains substantially full biological activity and has increased in vivo half-life compared to a composition of native t-PA.

7. The use according to claim 5 or claim 6 wherein the t-PA contains an amino acid other than asparagine at position 117.

8. The use according to claim 5 or claim 6 wherein the t-PA contains an amino acid other than serine or threonine at position 119.

9. The use according to claim 5 or claim 6 wherein the t-PA contains glutamine at position 117.

10. The use according to claim 5 or claim 6 wherein the t-PA contains methionine at position 119.

11. The use according to claim 5 or claim 6 wherein the t-PA contains proline at position 118.

12. The use according to any one of claims 5 to 11 wherein the t-PA is in single chain form.

13. The use according to claim 12 wherein the t-PA has glutamic acid at position 275.

14. The use according to claim 12 wherein the t-PA contains glutamine at position 117 and glutamic acid at position 275.

15. The use according to any one of claims 5 to 14 wherein the t-PA is produced via expression of recombinant DNA encoding said human t-PA having a codon at position 117 other than one encoding the amino acid asparagine or a codon at position 119 other than one encoding the amino acid serine or threonine.

16. The use in the preparation of a pharmaceutical composition of t-PA obtainable by the method of any one of claims 1 to 4.

## Patentansprüche (Patentansprüche für folgende(n) Vertragsstaat(en): BE, CH, DE, FR, GB, IT, LI, LU, NL, SE)

1. Verfahren zur Herstellung von menschlichem Gewebe-Plasminogenaktivator (t-PA), der im Vergleich zu nativem t-PA im wesentlichen die volle biologische Aktivität beibehalten hat und in vivo erhöhte Halbwertszeit aufweist, welches Verfahren die Behandlung von t-PA mit Endoglykosidase umfaßt, welche die funktionelle Kohlenhydratstruktur eliminiert, die in nativem t-PA bei Aminosäurerest 117 auftritt, mit der allerdings die funktionell unmodifizierten Kohlenhydratstrukturen bei den Aminosäureresten 184 und 448 beibehalten werden.

2. Verfahren nach Anspruch 1, worin der t-PA in einkettiger Form vorliegt.

3. Verfahren nach Anspruch 2, worin die Aminosäuresequenz so mutiert wird, daß die Spaltstelle zwischen den Aminosäureresten 275 und 276 zerstört wird.

4. Verfahren nach Anspruch 3, worin der t-PA an Position 275 einen Glutaminsäurerest aufweist.

5. Verwendung von menschlichem t-PA, der (a) eine Mutation aufweist, welche die Glykosylierungsstelle nur bei Aminosäurerest 117-119 zerstört, und (b) gegebenenfalls eine Mutation aufweist, um die Spaltstelle bei den Aminosäureresten 275-276 zu eliminieren, zur Herstellung einer pharmazeutischen Zusammensetzung, worin die Zusammensetzung im Vergleich zu einer Zusammensetzung mit nativem t-PA im wesentlichen die volle biologische Aktivität beibehält und in vivo erhöhte Halbwertszeit aufweist.

6. Verwendung von menschlichem t-PA, (a) dem funktionelle Kohlenhydratstrukturen an Aminosäurerest 117 fehlen, (b) der ansonsten funktionell unmodifizierte Kohlehydratstrukturen an den Aminosäureresten 184 und 448 aufweist, und (c) der gegebenenfalls eine Mutation aufweist, um die Spaltstelle an den Aminosäureresten 275-276 zu eliminieren, bei der Herstellung einer pharmazeutischen Zusammensetzung, worin die Zusammensetzung im Vergleich zu einer Zusammensetzung mit nativem t-PA im wesentlichen die volle biologische Aktivität beibehält und in vivo erhöhte Halbwertszeit aufweist.

7. Verwendung nach Anspruch 5 oder 6, worin der t-PA an Position 117 eine andere Aminosäure als Asparagin aufweist.

8. Verwendung nach Anspruch 5 oder 6, worin der t-PA an Position 119 eine andere Aminosäure als Serin oder Threonin aufweist.

9. Verwendung nach Anspruch 5 oder 6, worin der t-PA an Position 117 Glutamin aufweist.

10. Verwendung nach Anspruch 5 oder 6, worin der t-PA an Position 119 Methionin aufweist.

11. Verwendung nach Anspruch 5 oder 6, worin der t-PA an Position 118 Prolin aufweist.

12. Verwendung nach einem der Ansprüche 5 bis 11, worin der t-PA in einkettiger Form vorliegt.

13. Verwendung nach Anspruch 12, worin der t-PA an Position 275 Glutaminsäure aufweist.

14. Verwendung nach Anspruch 12, worin der t-PA an Position 117 Glutamin und an Position 275 Glutaminsäure aufweist.

15. Verwendung nach einem der Ansprüche 5 bis 14, worin der t-PA über rekombinante DNA-Expression von rekombinanter DNA erzeugt wird, die für den menschlichen t-PA kodiert und an Position 117 ein Codon aufweist, das nicht for die Aminosäure Asparagin kodiert, oder an Position 119 ein Codon aufweist, das nicht für die Aminosäuren Serin oder Threonin kodiert.

16. Pharmazeutische t-PA-Zusammensetzung, die im wesentlichen die volle biologische Aktivität beibehalten hat und in vivo erhöhte Halbwertszeit aufweist, umfassend menschlichen t-PA nach einem der Ansprüche 1 bis 15 in Kombination mit einem pharmazeutisch annehmbaren Träger.

## Patentansprüche (Patentansprüche für folgende(n) Vertragsstaat(en): AT, ES, GR)

1. Verfahren zur Herstellung von menschlichem Gewebe-Plasminogenaktivator (t-PA), der im Vergleich zu nativem t-PA im wesentlichen die volle biologische Aktivität beibehalten hat und in vivo erhöhte Halbwertszeit aufweist, welches Verfahren die Behandlung von t-PA mit Endoglykosidase umfaßt, welche die funktionelle Kohlenhydratstruktur eliminiert, die in nativem t-PA bei Aminosäurerest 117 auftritt, mit der allerdings die funktionell unmodifizierten Kohlenhydratstrukturen bei den Aminosäureresten 184 und 448 beibehalten werden.

2. Verfahren nach Anspruch 1, worin der t-PA in einkettiger Form vorliegt.

3. Verfahren nach Anspruch 2, worin die Aminosäuresequenz so mutiert wird, daß die Spaltstelle zwischen den Aminosäureresten 275 und 276 zerstört wird.

4. Verfahren nach Anspruch 3, worin der t-PA an Position 275 einen Glutaminsäurerest aufweist.

5. Verwendung von menschlichem t-PA, der (a) eine Mutation aufweist, welche die Glykosylierungsstelle nur bei Aminosäurerest 117-119 zerstört, und (b) gegebenenfalls eine Mutation aufweist, um die Spaltstelle bei den Aminosäureresten 275-276 zu eliminieren, zur Herstellung einer pharmazeutischen Zusammensetzung, worin die Zusammensetzung im Vergleich zu einer Zusammensetzung mit nativem t-PA im wesentlichen die volle biologische Aktivität beibehält und in vivo erhöhte Halbwertszeit aufweist.

6. Verwendung von menschlichem t-PA, (a) dem funktionelle Kohlenhydratstrukturen an Aminosäurerest 117 fehlen, (b) der ansonsten funktionell unmodifizierte Kohlehydratstrukturen an den Aminosäureresten 184 und 448 aufweist, und (c) der gegebenenfalls eine Mutation aufweist, um die Spaltstelle an den Aminosäureresten 275-276 zu eliminieren, bei der Herstellung einer pharmazeutischen Zusammensetzung, worin die Zusammensetzung im Vergleich zu einer Zusammensetzung mit nativem t-PA im wesentlichen die volle biologische Aktivität beibehält und in vivo erhöhte Halbwertszeit aufweist.

7. Verwendung nach Anspruch 5 oder 6, worin der t-PA an Position 117 eine andere Aminosäure als Asparagin aufweist.

8. Verwendung nach Anspruch 5 oder 6, worin der t-PA an Position 119 eine andere Aminosäure als Serin oder Threonin aufweist.

9. Verwendung nach Anspruch 5 oder 6, worin der t-PA an Position 117 Glutamin aufweist.

10. Verwendung nach Anspruch 5 oder 6, worin der t-PA an Position 119 Methionin aufweist.

11. Verwendung nach Anspruch 5 oder 6, worin der t-PA an Position 118 Prolin aufweist.

12. Verwendung nach einem der Ansprüche 5 bis 11, worin der t-PA in einkettiger Form vorliegt.

13. Verwendung nach Anspruch 12, worin der t-PA an Position 275 Glutaminsäure aufweist.

14. Verwendung nach Anspruch 12, worin der t-PA an Position 117 Glutamin und an Position 275 Glutaminsäure aufweist.

15. Verwendung nach einem der Ansprüche 5 bis 14, worin der t-PA über rekombinante DNA-Expression von rekombinanter DNA erzeugt wird, die für den menschlichen t-PA kodiert und an Position 117 ein Codon aufweist, das nicht für die Aminosäure Asparagin kodiert, oder an Position 119 ein Codon aufweist, das nicht für die Aminosäuren Serin oder Threonin kodiert.

16. Verwendung von t-PA, der nach dem Verfahren nach einem der Ansprüche 1 bis 4 erhältlich ist, zur Herstellung einer pharmazeutischen Zusammensetzung.

## Revendications (Revendications pour l'(les) Etat(s) contractant(s) suivant(s): BE, CH, DE, FR, GB, IT, LI, LU, NL, SE)

1. Procédé de préparation d'activateur tissulaire du plasminogène humain (t-PA) ayant conservé l'essentiel de son activité biologique et présentant une demi-vie in vivo prolongée par rapport au t-PA natif, comprenant le traitement du t-PA avec une endoglycosidase qui élimine la structure glucidique fonctionnelle portée par le résidu d'acide aminé 117 dans le t-PA natif, mais conserve des structures glucidiques fonctionnelles intactes au niveau des résidus d'acides aminés 184 et 448.

2. Procédé selon la revendication 1 dans lequel le t-PA est sous forme de chaîne unique.

3. Procédé selon la revendication 2 dans lequel la séquence d'acides aminés fait l'objet de mutation en vue de détruire le site de clivage entre les résidus d'acides aminés 275 et 276.

4. Procédé selon la revendication 3 dans lequel le t-PA comporte un résidu d'acide glutamique en position 275.

5. Utilisation dans la préparation d'une composition pharmaceutique de t-PA humain (a) comportant une mutation qui détruit le site de glycosylation uniquement au niveau des résidus d'acides aminés 117-119, et (b) éventuellement une mutation destinée à éliminer le site de clivage au niveau des résidus d'acides aminés 275-276, utilisation dans laquelle la composition conserve l'essentiel de son activité biologique et présente une demi-vie in vivo prolongée par rapport à une composition de t-PA natif.

6. Utilisation dans la préparation d'une composition pharmaceutique de t-PA humain (a) dépourvu de structure glucidique fonctionnelle au niveau du résidu d'acide aminé 117, (b) présentant par ailleurs des structures glucidiques fonctionnelles intactes au niveau des résidus d'acides aminés 184 et 448, et (c) éventuellement une mutation destinée à éliminer le site de clivage au niveau des résidus d'acides aminés 275-276, utilisation dans laquelle la composition conserve l'essentiel de son activité biologique et présente une demi-vie in vivo prolongée par rapport à une composition de t-PA natif.

7. Utilisation selon la revendication 5 ou la revendication 6 dans laquelle le t-PA contient un acide aminé autre que l'asparagine en position 117.

8. Utilisation selon la revendication 5 ou la revendication 6 dans laquelle le t-PA contient un acide aminé autre que la sérine ou la thréonine en position 119.

9. Utilisation selon la revendication 5 ou la revendication 6 dans laquelle le t-PA contient une glutamine en position 117.

10. Utilisation selon la revendication 5 ou la revendication 6 dans laquelle le t-PA contient une méthionine en position 119.

11. Utilisation selon la revendication 5 ou la revendication 6 dans laquelle le t-PA contient une proline en position 118.

12. Utilisation selon l'une quelconque des revendications 5 à 11 dans laquelle le t-PA est sous forme de chaîne unique.

13. Utilisation selon la revendication 12 dans laquelle le t-PA comporte un acide glutamique en position 275.

14. Utilisation selon la revendication 12 dans laquelle le t-PA comporte une glutamine en position 117 et un acide glutamique en position 275.

15. Utilisation selon l'une quelconque des revendications 5 à 14 dans laquelle le t-PA est produit par voie recombinante qui repose sur l'expression d'un ADN codant pour ledit t-PA humain comportant un codon en position 117 ne codant pas toutefois pour l'acide aminé asparagine ou un codon en position 119 ne codant pas toutefois pour l'acide aminé sérine ou thréonine.

16. Composition pharmaceutique à base de t-PA ayant conservé l'essentiel de son activité biologique et présentant une demi-vie in vivo prolongée, comprenant un t-PA humain tel que défini dans l'une quelconque des revendications 1 à 15 en mélange avec un véhicule pharmaceutiquement acceptable.

## Revendications (Revendications pour l'(les) Etat(s) contractant(s) suivant(s): AT, ES, GR)

1. Procédé de préparation d'activateur tissulaire du plasminogène humain (t-PA) ayant conservé l'essentiel de son activité biologique et présentant une demi-vie in vivo prolongée par rapport au t-PA natif, comprenant le traitement du t-PA avec une endoglycosidase qui élimine la structure glucidique fonctionnelle portée par le résidu d'acide aminé 117 dans le t-PA natif, mais conserve des structures glucidiques fonctionnelles intactes au niveau des résidus d'acides aminés 184 et 448.

2. Procédé selon la revendication 1 dans lequel le t-PA est sous forme de chaîne unique.

3. Procédé selon la revendication 2 dans lequel la séquence d'acides aminés fait l'objet de mutation en vue de détruire le site de clivage entre les résidus d'acides aminés 275 et 276.

4. Procédé selon la revendication 3 dans lequel le t-PA comporte un résidu d'acide glutamique en position 275.

5. Utilisation dans la préparation d'une composition pharmaceutique de t-PA humain (a) comportant une mutation qui détruit le site de glycosylation uniquement au niveau des résidus d'acides aminés 117-119, et (b) éventuellement une mutation destinée à éliminer le site de clivage au niveau des résidus d'acides aminés 275-276, utilisation dans laquelle la composition conserve l'essentiel de son activité biologique et présente une demi-vie in vivo prolongée par rapport à une composition de t-PA natif.

6. Utilisation dans la préparation d'une composition pharmaceutique de t-PA humain (a) dépourvu de structure glucidique fonctionnelle au niveau du résidu d'acide aminé 117, (b) présentant par ailleurs des structures glucidiques fonctionnelles intactes au niveau des résidus d'acides aminés 184 et 448, et (c) éventuellement une mutation destinée à éliminer le site de clivage au niveau des résidus d'acides aminés 275-276, utilisation dans laquelle la composition conserve l'essentiel de son activité biologique et présente une demi-vie in vivo prolongée par rapport à une composition de t-PA natif.

7. Utilisation selon la revendication 5 ou la revendication 6 dans laquelle le t-PA contient un acide aminé autre que l'asparagine en position 117.

8. Utilisation selon la revendication 5 ou la revendication 6 dans laquelle le t-PA contient un acide aminé autre que la sérine ou la thréonine en position 119.

9. Utilisation selon la revendication 5 ou la revendication 6 dans laquelle le t-PA contient une glutamine en position 117.

10. Utilisation selon la revendication 5 ou la revendication 6 dans laquelle le t-PA contient une méthionine en position 119.

11. Utilisation selon la revendication 5 ou la revendication 6 dans laquelle le t-PA contient une proline en position 118.

12. Utilisation selon l'une quelconque des revendications 5 à 11 dans laquelle le t-PA est sous forme de chaîne unique.

13. Utilisation selon la revendication 12 dans laquelle le t-PA comporte un acide glutamique en position 275.

14. Utilisation selon la revendication 12 dans laquelle le t-PA comporte une glutamine en position 117 et un acide glutamique en position 275.

15. Utilisation selon l'une quelconque des revendications 5 à 14 dans laquelle le t-PA est produit par voie recombinante qui repose sur l'expression d'un ADN codant pour ledit t-PA humain comportant un codon en position 117 ne codant pas toutefois pour l'acide aminé asparagine ou un codon en position 119 ne codant pas toutefois pour l'acide aminé sérine ou thréonine.

16. Utilisation dans la préparation d'une composition pharmaceutique de t-PA pouvant être obtenu par le procédé selon l'une quelconque des revendications 1 à 4.
